# EUROPEAN PATENT APPLICATION

(11) **EP 1 849 485 A1**
(43) Date of publication of application: **31.10.2007**
(21) Application number: 06008646.9
(22) Date of filing: 26.04.2006
(51) Int. Cl.: A61L 9/04, A61L 9/14

(54) **Discharge device and method for evaporating a liquid and evaporator**

(71) Applicant: Boehringer Ingelheim microParts GmbH, 44227 Dortmund (DE)
(72) Inventor: Blankenstein, Gert, 44141 Dortmund (DE); Peters, Ralf-Peter, 51467 Bergisch-Gladbach (DE)
(74) Representative: Gesthuysen, von Rohr & Eggert

(57) **Abstract**

A discharge device, an evaporator and a method are proposed for evaporating a liquid to the atmosphere. The liquid is supplied to an evaporation only by means of a capillary.

## Description

The present invention relates to a discharge device for evaporating a liquid, to an evaporator for evaporating a liquid and to a method for evaporating a liquid to the atmosphere.

In particular, the present invention relates to the dispensing of any active ingredient such as fragrances, perfumes, air fresheners, pharmaceuticals or the like, preferably in enclosed spaces.

Many continuous liquid delivery devices are on the market or have been proposed. There are two main types, namely passive and active ones. In passive devices, a liquid is usually absorbed, deluted or dissolved in a carrier such as a gel, foam or liquid solvent. In such passive devices, the transfer of the liquid or any active ingredient to the atmosphere depends in particular on the rate of evaporation which is dependent on room temperature and the rate of air circulation, and on other factors. Active devices are more complicated and usually use gas pressure or any other active drive.

US 2002/0168301 A1, which forms the starting point of the present invention, discloses a passive discharge device for evaporating a liquid to the atmosphere. The device comprises a dip tube for guiding the liquid to an evaporation surface. The liquid is contained in a closed container, and the liquid is pressurized and fed only by thermal expansion of air and/or liquid in the container, The discharge rate depends on temperature variations. The device does not ensure a defined, continous discharge and evaporation of the liquid.

Object of the present invention is to provide a passive discharge device and a method for evaporating a liquid as well as an evaporator, wherein a more uniform and/or continuous release and evaporation of liquid is possible, in particular wherein any dependency on temperature variations, room temperature and/or air circulation can be avoided or at least reduced and wherein any periodic, direct or indirect activation by a user or by any electrical device is not necessary.

The above object is achieved by a discharge device according to claim 1, an evaporator according to claim 14 or a method according to claim 20. Preferred embodiments are subject of the subclaims.

A basic idea of the present invention is to use the capillary effect to generate the desired, in particular precise flow rate of the preferably non-pressurized liquid from a container to evaporator. Further, a flow restriction device may be provided that determines the flow rate and, thus, the actual rate of evaporation. Therefore, the dependency of the evaporation rate on room temperature, air circulation or the like can be avoided or at least reduced. Preferably, the flow restriction device comprises at least one channel, in particular a long capillary channel that restricts the flow of liquid as desired.

In the present invention, the term "liquid" has to be understood in a broad sense. In particular, it shall cover all kinds of ingredients, liquids, fluids, mixtures, suspensions, liquefied gases, or the like that may be evaporated. Preferably, the liquid is or contains an oil, a solvent, a fragrance, a perfume, an air freshener, a pharmaceutical, a therapeutic or any other active ingredient or the like.

A further aspect of the present invention is directed to an evaporator for evaporating the liquid. The evaporator comprises an evaporation surface which is designed preferably by micro-structuring such that the surface area is increased and/or the liquid forms an essential uniform film on the evaporation surface. Thus, the dependency of the evaporation rate on room temperature, air circulation or the like can be avoided or at least reduced.

Further aspects, advantages and features of the present invention will be apparent from the claims and following detailed description of preferred embodiments. In the drawings show:
- Fig. 1: a schematic section of a discharge device according to a first embodiment;
- Fig. 2: a schematic section of a discharge device with a flow restriction device and an evaporator according to a second embodiment;
- Fig. 3: a schematic section of the flow restriction device of the second embodiment;
- Fig, 4: a schematic view of an evaporator of the discharge device according to the second embodiment;
- Fig. 5: a schematic perspective section of a part of a discharge device with a flow restriction device according to a third embodiment;
- Fig. 6: a perspective view of the flow restriction device according to the third embodiment;
- Fig. 7: a perspective view of a flow restriction device according to a fourth embodiment;
- Fig. 8: a partial perspective view of an evaporator according to a fifth embodiment; and

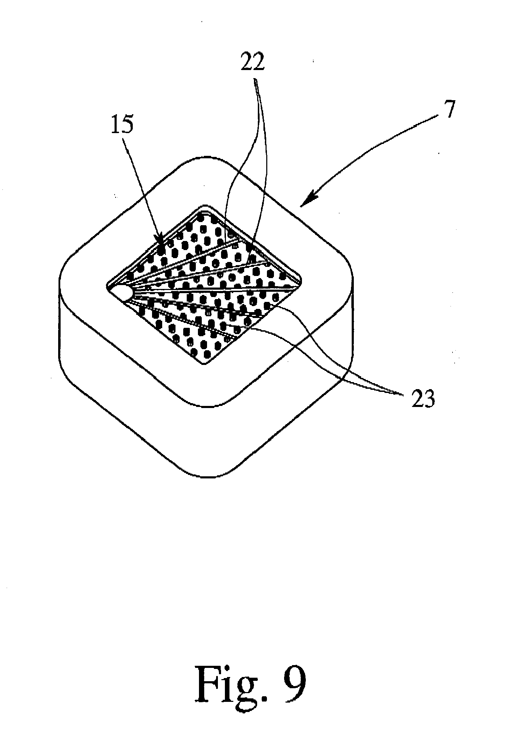

- Fig. 9: a perspective view of an evaporator according to a sixth embodiment.

In the figures, the same reference signs are used for same or similar components, wherein same or similar characteristics or advantages are achieved even if a repeated discussion is omitted.

Fig, 1 shows a schematic section of a discharge device D according to a first embodiment of the present invention. The discharge device D comprises a container 1 with a liquid 2. The liquid 2 comprises preferably an active ingredient, a solvent and/or a liquid and compressed gas. Reference is made to the above understanding of the term "liquid". The liquid 2 may be placed in a bag (not shown) in the container 1.

The discharge device D comprises a capillary 4 and an evaporator 7. The capillary 4 feeds (pressurizes) the liquid 2 by capillary forces only, preferably directly to the evaporator 7, Due to the capillary effect or force a continuous supply or feeding of liquid 2 can be achieved that is highly independent on evaporation rate, temperature, air circulation or the like.

Preferably, the capillary 4 forms a dip tube, i.e. extends into the liquid 2 and/or to the bottom of the container 1.

Preferably, the dip tube / capillary 4 reduces or restricts the flow rate of the liquid 2 sufficiently and, thus, forms a restriction device.

The dip tube / capillary 4 may be flexible and/or designed as a hose or may be rigid.

Preferably, the capillary 4 has a very small diameter, in particular of less than 1 mm, preferably about 0,1 mm to 0,8 mm, or less in order to generate a high capillary force, but low flow rate.

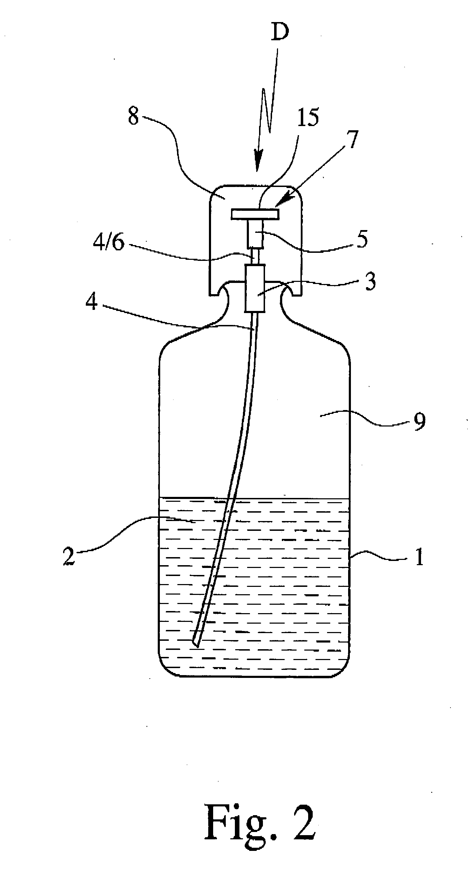

Fig. 2 shows a second embodiment of the discharge device D which comprises optionally a valve 3 that is preferably mounted at the top of the container 1 and connected to the capillary 4.

The valve 3 is preferably of the on/off-type. However, the valve 3 can also be designed such that it can be only opened once. In this case, the valve 3 can be formed by a removable lid, cap or the like. Preferably, the valve 3 can be locked in its open and/or closed state.

The discharge device D may further comprise a (separate) flow restriction device 5 which is preferably directly or indirectly connected fluidically with the capillary 4. Preferably, the flow restriction device 5 is placed after the optional valve 3. However, it is also possible to place the flow restriction device 5 before the valve 3 and/or to integrate the flow restriction device 5 into the valve 3.

The evaporator 7 is fluidically connected to the outlet of the capillary 4 or - in the present embodiment - via the flow restriction device 5 for supply with liquid 2 for evaporation.

The discharge device D comprises optionally an actuator 8. The actuator 8 may be mounted on the container 1 and/or the valve 3 in particular such that the valve 3 can be opened by pressing down the actuator 8. Preferably, the actuator 8 is designed in such a way that once valve 3 is opened it stays open after the user ceases to press the actuator 8, This can be achieved by a ratchet mechanism, a locking mechanism or the like.

Preferably, the actuator 8 has a locking mechanism that allows the user to turn valve 3 on and leave it in the open position. The locking mechanism may lock the valve 3 in the open position permanently or may have an on/off-feature.

It has to be noted that the flow restriction device 5 can also be located in or integrated into the actuator 8.

The capillary 4 and/or flow restriction device 5 may (further) restrict the flow rate of liquid 2 from the container 1 to the evaporator 7 in the open state of the valve 3 below or substantially equal to the possible rate of evaporation of the liquid 2 by the evaporator 7. Thus, the valve 3 can be opened permanently for continuous release from the container 1 and evaporation of the liquid 2 by the evaporator 7.

The capillary 4 and/or flow restriction device 5 preferably restricts the flow rate of liquid 2 such that the flow rate is 0.01 to 2.0 g/d (grams per day), most preferably 0.05 to 0.5 g/d. This is a relatively low, reasonable range suitable for most applications, in particular for air fresheners or the like.

The useable lifetime of the discharge device D is preferably between 2 and 36 weeks, i.e. with permanently opened valve 3. With closed valve 3, the discharge device D can be stored for at least more than one year,

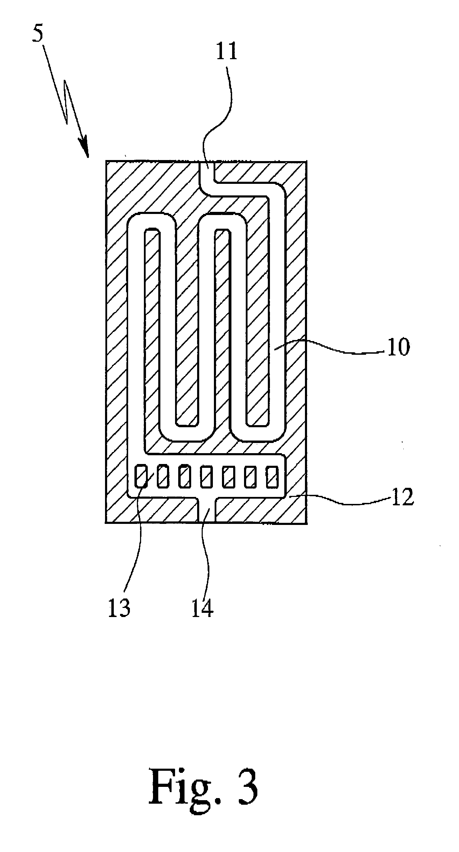

According to the most preferred embodiment, the capillary 4 and/or flow restriction device 5 comprises at least one throttle channel 10, preferably a long capillary tube or channel 10. Fig. 3 shows a schematic section of the flow restriction device 5 as an example.

The required length and diameter of the channel 10 can be calculated by using the classical laminar flow equations once the flow rate, pressure and viscosity and density of the liquid 2 are known. The shorter the length of the channel 10 the smaller is the hydraulic diameter required for any given flow rate and set of physical parameters.

The diameter should be as large as possible to minimize clogging or blocking. Preferably, the average or hydraulic diameter of the capillary and/or channel 10 is between 1 µm and 1 mm, more preferably between 50 and 200 µm, in particular between 75 and 125 µm. The cross section of the channel 10 may have any suitable form and does not have to be necessarily circular.

The length is also a factor determining the flow resistance and, thus, the flow rate, preferably, the length of the capillary and/or channel 10 is between 1 mm and 10 m, more preferably between 10 mm and 1 m.

The capillary 4 and/or channel 10 may take the shape of a meander. However, the capillary and/or channel 10 may also be essentially straight or take the shape of a spiral.

In a further embodiment, the channel 10 has or forms a portion with higher capillary forces, in particular due to a reduced diameter or cross section, in order to avoid that the capillary 3 and/or channel 10 empties completely when the evaporation rate is much higher than the flow rate. This portion (not shown) is preferably formed near the outlet of the capillary 4 and/or fuel restriction device 5 and/or of the channel 10.

According to a further embodiment (not shown), the discharge device 4 may comprise multiple capillaries 4 connected in parallel and/or the flow restriction device 5 comprises multiple channels 10 connected in parallel. The use of the respective capillaries 4 or channels 10 is preferably variable (at least one capillary 4 or channels 10 can be individually blocked) for changing the flow rate. In particular, this arrangement may form a regulating device, wherein the capillaries 4 or channels 10 can be opened sequentially as desired. Preferably, the user may switch from one flow rate to at least one other flow rate by pressing a button, turning the actuator 8, operating any other element or the like.

Thus, the flow rate is adjustable. However, there are also other possibilities that can be used to adjust the flow rate, in particular by varying the effective length or diameter of the capillary 4 and/or channel 10 and/or by additional measures, like a throttle valve (not shown) or the like.

Preferably, the flow restriction device 5 comprises a molded body 12, preferably made of plastic, as shown in fig. 2, which forms the channel(s) 10 and optionally a filter 13 upstream of the channel 10. The structured body 12 and/or the channel 10 or any other flow restriction structure can be made of any suitable material and/or structured with any other suitable method other than moulding.

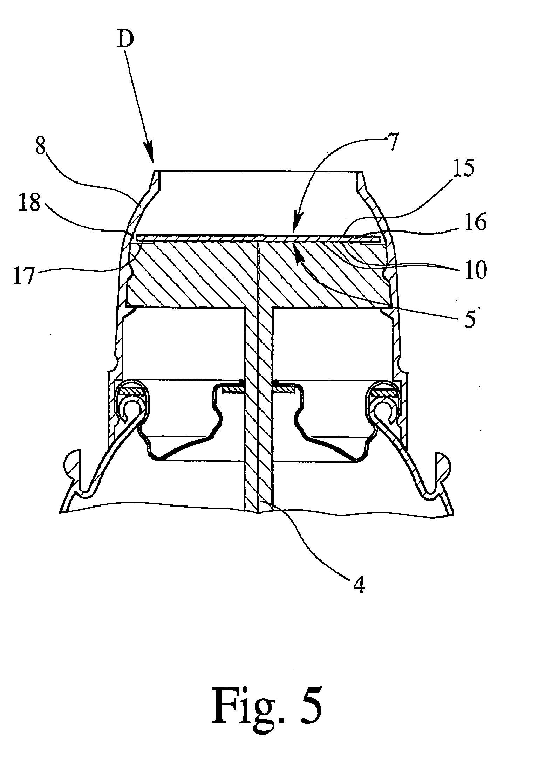

The structured body 12 is preferably covered by a lid, film or any other suitable covering (e.g. covering 16 shown in Fig. 5), so that the liquid 2 supplied by the stem 6 can only enter into the flow restriction device 5 / the filter 13 via the inlet 14 and leave the flow restriction device 5 via the outlet 11, wherein evaporation of the liquid 2 is prevented in the flow restriction device 5. Preferably, the molded body 12 is sealed by heatsealing a film or the like on the surface of the body 12 or by ultrasonically welding a second plastic moulding, cover or the like over the surface forming a passageway, i.e, at least the channel 10 and optionally the filter 13, with the molded body 12.

The filter 13 prevents blocking or clogging of the channel 10. Preferably, the filter 13 has filter bores or openings of smaller size than the diameter of the subsequent channel(s) 10 to filter out any problematic particles in the liquid 2.

In the second embodiment, the filter 13 is integrated into the flow restriction device 5 and/or the body 12. However, the filter 13 can also be made and/or arranged separately from the flow restriction device 5. For example, the filter 13 could be integrated into the stem 6 or the valve 3. In any case, the filter 13 is preferably arranged upstream in series with the flow restriction device 5 or at least its channel 10.

According to another embodiment (not shown), the flow restriction device 5 may comprise additionally or alternatively at least one restriction orifice, preferably with a hydraulic diameter of 30 to 100 µm, in order to reduce or restrict the flow rate of the liquid 2 as desired. The advantage of the restriction orifice arrangement over the channel arrangement is its overall smaller size. The disadvantage is its higher susceptibility to blockage.

The evaporator 7 is fluidically connected to the flow restriction device 5, in particular to its outlet 11, The construction of the evaporator 7 will be discussed in more detail with reference to the other figures and embodiments.

In the first embodiment, the flow restriction device 5 and the evaporator 7 are preferably arranged adjacent to each other, in particular one above the other. It is also possible to integrate the flow restriction device 5 into the evaporator 7 or vice versa. Alternatively or additionally, the evaporator 7 may be integrated into the actuator 8 of the discharge device D.

The evaporator 7 may comprise a plastic plate with molded grooves, a sponge like material, adsorbent paper or a conical cup or any other device that can hold liquid 2 while it evaporates, It is preferably placed within the actuator 8 and protected with a cap, cover, screen or the actuator 8 to prevent users coming into direct contact with the liquid 2. A total exposed area of the evaporator 7 is large enough to evaporate the liquid 2 at a rate at least substantially equal or larger than the flow rate of liquid 2 through the flow restriction device 5.

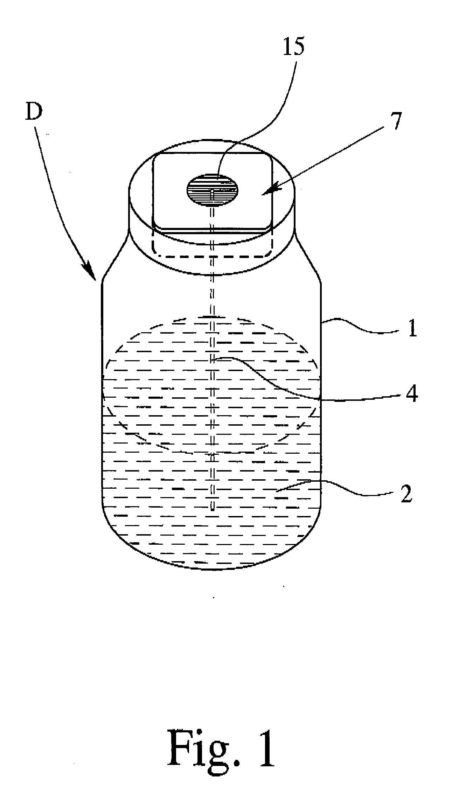

According to the present invention, the evaporator 7 for evaporating the liquid 2 comprises an evaporation surface 15 (as indicated in fig. 1), which is designed such that the surface area is increased and/or the liquid 2 forms an essentially uniform film on the evaporation surface 15. Preferably, the evaporation surface 15 is micro-structured to achieve these properties.

In the following, further embodiments of the present invention are described with reference to the further fig., wherein only essential differences will be emphasized. Thus, the above explanation applies in addition as well.

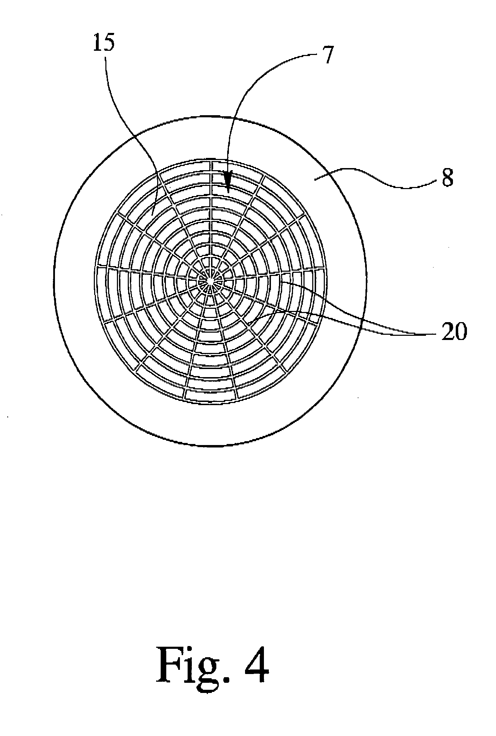

Fig. 4 shows a spider-net-like structure of grooves 20 on the evaporation surface 15. These grooves 20 or similar structures promote the forming of a uniform film of liquid 2 on the evaporation surface 15. Further, a central supply channel for supply with fluid 2 from the floor restriction device 2 is shown.

Fig. 5 shows a third embodiment of the discharge device D. The flow restriction device 5 - in particular its channel 10 in spiral form - is arranged substantially horizontal and essentially parallel to the horizontal evaporation surface 15 of the evaporator 7 located above. In particular, the evaporator 7 forms a covering 16 of the capillary 4 or the flow restriction device 5 or, vice versa, the covering 16 of the floor restriction device 5 forms the evaporation surface 15 on its upper face.

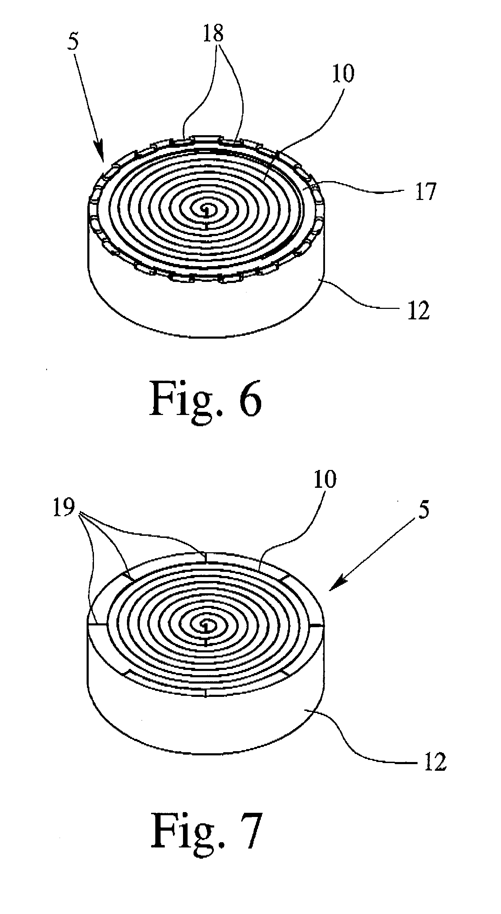

Fig. 6 shows the enlarged flow restriction device 5 without the covering 16. The preferred spiral form of the channel 10 is clearly visible. Further, a circumferential ring space 17 for liquid 2 is provided. This forms a liquid buffer. The radial depressions, notches or grooves 18 form either evaporation areas or a fluidic connection so that the liquid 2 can flow around the covering 16 and up to the evaporation surface 15.

Fig. 7 shows a forth embodiment of the flow restriction device 5 without covering 16 and without the associated evaporator 7. The spiral form of the channel 10 is clearly visible. Further, radial channel connections 19 are provided. Depending on the rotational position of the actuator 8 or the like at least one of the channel connections 19 can be connected with the evaporator 7 (not shown). The effective length of the channel 10 varies depending on the respectively connected channel connections 19. Thus, the flow rate of liquid 2 can be adjusted,

According to an alternative (not shown), at least two channels 10 forming two parallel spirals can be provided and connected in parallel or in series, as desired. Individually blocking can be used to vary the effective length to adjust the flow resistance and, thus, the flow rate.

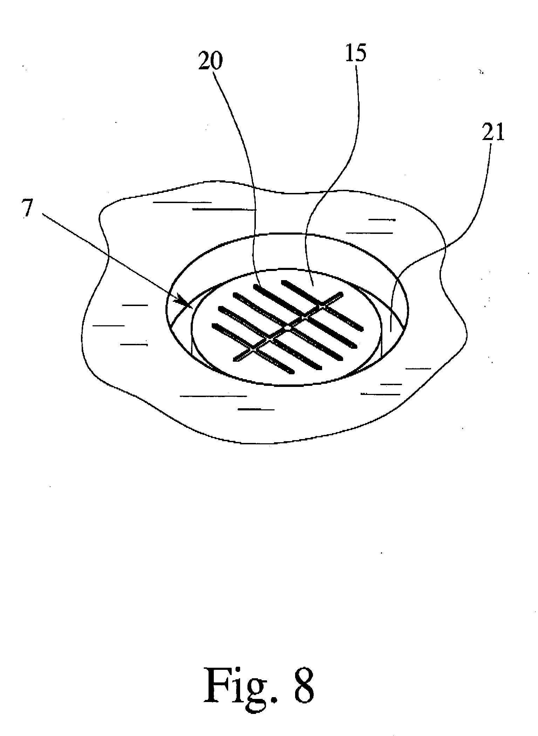

Fig. 8 shows a fifth embodiment of the evaporator 7. The evaporation surface 15 comprises a grid of grooves or recesses 20, These grooves, recesses 20 or similar structures promote the forming of a uniform film of liquid 2 on the evaporation surface 15. In addition, the surface 15 is surrounded by a circumferential groove 20 that is deeper so that is does not fill with liquid 2. This ring groove 21 forms an outer limit for the liquid 2 on the surface 15.

Fig, 9 shows a sixth embodiment of the evaporator 7. The evaporation surface 15 comprises another grid of grooves 22 and microstructures, like posts 23 or the like. These structures 23 increase the total surface area that is covered by the liquid 2 and, thus, increase the rate of evaporation.

The respective features and aspects of the different embodiments can be combined as disered or interchanged or used for other embodiments.

## Claims

1. Discharge device (D) for evaporating a liquid (2) comprising a container (1) with the preferably non-pressurized liquid (2), a capillary (4), and an evaporator (7), the capillary (4) feeding the liquid (2) to the evaporator (7).

2. Discharge device according to claim 1, **characterized in that** the capillary (4) forms a dip tube and/or is flexible.

3. Discharge device according to claim 1, **characterized in that** the capillary (4) is rigid.

4. Discharge device according to any one of the preceding claims, **characterized in that** the flow rate is 0.01 to 2.0 g/d, preferably 0.05 to 0.5 g/d.

5. Discharge device according to any one of the preceding claims, **characterized in that** the flow rate is adjustable.

6. Discharge device according to any one of the preceding claims, **characterized in that** the capillary (4) is essentially straight or takes the shape of a spiral or a meander.

7. Discharge device according to any one of the preceding claims, **characterized in that** the average or hydraulic diameter of the capillary (4) is between 1 µm and 0.9 mm, preferably between 50 and 200 µm, more preferably between 75 and 125 µm.

8. Discharge device according to any one of the preceding claims, **characterized in that** the length of the capillary (4) is between 1 mm and 10 m, preferably between 10 mm and 1 mm.

9. Discharge device according to any one of the preceding claims, **characterized in that** the effective length or diameter of the capillary (4) is variable for changing the flow rate.

10. Discharge device according to any one of the preceding claims, **characterized in that** the capillary (4) and/or a flow restriction device (5) is integrated into the evaporator (7).

11. Discharge device according to any one of the preceding claims, **characterized in that** the evaporator (7) is protected by a cap, cover, screen or actuator (8) and/or that the evaporator (7) is integrated into an actuator (8) of the discharge device (D).

12. Discharge device according to any one of the preceding claims, **characterized in that** the capillary (4) forms the only means or drive for feeding the liquid (2) to the evaporator (7).

13. Discharge device according to any one of the preceding claims, **characterized in that** the liquid (2) is or contains an oil, a solvent, a fragrance, a perfume, an air freshener, a pharmaceutical, a therapeutic or any other active ingredient and/or that the evaporator (7) is designed according to any one of claims 14 to 19.

14. Evaporator (7) for evaporating a liquid (2) with an evaporation surface (15) and with a capillary (4) preferably forming a dip tube for feeding the liquid (2) to the evaporation surface (10).

15. Evaporator according to claim 14, **characterized in that** the evaporation surface (15) is designed such that the surface area is increased and/or the liquid (2) forms an essentially uniform film on the evaporation surface (15) and/or is microstructured such that the surface area is increased and/or the liquid (2) forms an essentially uniform film on the evaporation surface (15).

16. Evaporator according to claim 14 or 15, **characterized in that** the evaporation surface (15) is essentially planar and/or formed by a structured or molded body (12), preferably made of plastic.

17. Evaporator according to any one of claims 14 to 16, **characterized in that** the liquid (2) is driven by capillary forces onto and/or on the evaporation surface (15).

18. Evaporator according to any one of claims 14 to 17, **characterized in that** the liquid (2) is or contains an oil, a solvent, a fragrance, a perfume, an air freshener, a pharmaceutical, a therapeutic or any other active ingredient.

19. Evaporator according to any one of claims 14 to 18, **characterized in that** the evaporation rate is 0.01 to 2.0 g/d, preferably 0.05 to 0.5 g/d.

20. Method for evaporating a liquid (2) to the atmosphere, wherein the liquid (2) is pressurized and preferably continuously discharged only by means of a capillary (4) to an evaporator (7) for evaporating the liquid (2).
